# EUROPÄISCHE PATENTSCHRIFT

(11) **EP 1 284 695 B1**
(45) Veröffentlichungstag und Bekanntmachung des Hinweises auf die Patenterteilung: **30.03.2005**
(21) Anmeldenummer: 01949220.6
(22) Anmeldetag: 30.05.2001
(51) Int. Cl.: A61F 5/02, A41D 13/00, A41D 13/05

(54) **ORTHOPÄDISCHE SCHIENE UND ORTHOPÄDISCHES KLEIDUNGSSTÜCK**
ORTHOPEDIC SPLINT AND ORTHOPEDIC GARMENT
ATTELLE ORTHOPEDIQUE ET ARTICLE VESTIMENTAIRE ORTHOPEDIQUE

(30) Priorität: 30.05.2000 DE 20009763 U
(43) Veröffentlichungstag der Anmeldung: 26.02.2003
(73) Patentinhaber: Medi Bayreuth Weihermüller & Voigtmann GmbH & Co. KG, 95448 Bayreuth (DE)
(72) Erfinder: MINNE, Helmut, W., 31812 Bad Pyrmont (DE)
(74) Vertreter: Schuhmann, Albrecht
(86) Internationale Anmeldenummer: PCT/DE2001/002053
(87) Internationale Veröffentlichungsnummer: WO 2001/091676

(56) Entgegenhaltungen:
- DE-A- 3 319 053
- DE-U- 29 507 787
- DE-U- 29 911 206
- US-A- 5 400 801
- US-A- 5 768 717

## Beschreibung

Die vorliegende Erfindung betrifft eine steife Schiene mit den Merkmalen des Oberbegriffs des Anspruchs 1, welche sich speziell für Patienten mit Osteoporose-Beschwerden eignet.

Der Einbruch eines Wirbelkörpers ist eine typische Spätkomplikation der Osteoporose. Die entstehende Deformierung des Wirbelkörpers führt zu Fehlstellungen der Zwischenwirbelgelenke, Fehlfunktionen der Sehnen, Bänder und Muskulatur. Dies wird zur Ursache für chronischen Schmerz, für Behinderungen im Alltag.

Aufgabe einer Orthese ist es dann, die Wirbelsäule wieder aufzurichten, um den gequetschten Wirbelkörper, hierdurch die gezerrte und schmerzende Knochenhaut zu entlasten, die Zwischenwirbelgelenkfunktion zu verbessern und hierdurch dauernden Gelenkschaden zu verhindern. Dies verbessert die Behandlungsmöglichkeiten nach frischer Fraktur und bei chronischer Wirbelsäulendeformierung. Die vorliegende Erfindung erlaubt diese Aufrichtung durch Anmodellieren der gekrümmten Wirbelsäule an eine Orthese, die sich von hinten an den Rücken anlegt. Sie vermag, hierdurch Schmerzauslösung zu verhindern, die Mobilisierung zu verbessern.

Alle bisher mit diesem Behandlungsziel eingesetzten Orthesen erstreben dieses Ziel durch Einschnüren (elastische Bauchbinden) oder Druckauslösung auf Strukturen, die als Widerlager zur Aufrichtung genutzt werden. Dies engt die Patienten ein, behindert ihre Beweglichkeit in zum Teil unerträglichen Maße und führt dazu, daß diese Hilfsinstrumente nicht in wünschenswertem Umfang genutzt werden. Dies wird hierdurch in der Regel zum Hindernis für eine angemessene Nutzung eines an sich wünschenswerten therapeutischen Ansatzes.

Aus der DE-U-297 20 475 ist eine Osteoporose-Minimalorthese bekannt, die einerseits vermag, die Wirbelsäule wünschenswert aufzurichten, jedoch konstruktionsbedingt, ohne die Brust- und Bauchatmung zu behindern und ohne die Beweglichkeit im Schulter- und Armbereich einzuschränken. Dies hat die Akzeptanz dieser neuen Orthese durch den Patienten gesteigert und verbessert hierdurch deren Behandelbarkeit eindrucksvoll. Die Behandelbarkeit von Patienten mit Osteoporose und Wirbelkörpereinbrüchen wird optimiert.

Als nachteilig wird aber hier, wie bei den meisten Arten der vorgenannten Hilfsinstrumente, empfunden, daß diese unter der Kleidung "auftragen" und als solche sichtbar, bzw. wahrnehmbar sind.

Bekannt ist weiter aus der EP-A-0 941 721 ein Geradehalter mit einer Osteoporose-Orthese, bei dem ein System von Bändern und Bandagen eine Haltefunktion erfüllen und auch zu einer Kräftigung der Muskulatur führen können. Dieses Hilfsmittel ist zweiteilig und nicht als Kleidungsstück ausgeführt und stützt nicht speziell die Wirbelsäule. Bekannt sind auch Mieder oder Korsetts mit Stützstangen oder Gestellen (DE-U-89 04 637, DE-A-32 22 168, DE-A-32 32 638).

Aufgabe der vorliegenden Erfindung ist es, eine Schiene zur Stützung der Wirbelsäule und insbesondere ein orthopädisches Kleidungsstück zu schaffen, das die Optik als "normales" Kleidungsstück weitgehend bewahrt, das jedoch sehr effektiv zur Behandlung der Osteoporose als Stütze für die Wirbelsäule wirkt.

Diese Aufgabe wird durch eine Schiene mit den in Anspruch 1 genannten Merkmalen gelöst. Die Schiene weist eine Aussparung für die Dornfortsätze der Wirbelsäule auf, was zu einer Entlastung der druckempfindlichen Dornfortsätze führt. Neben den Dornfortsätzen ist sie an den Körper des Patienten anmodelliert. Im Rumpfbereich weist sie einen vergrößerten Durchmesser ihres Querschnitts auf, wodurch die Auflagefläche vergrößert wird und der Druck pro Auflagefläche vermindert wird. Die erfindungsgemäße Schiene ermöglicht orthopädische Kleidungsstücke wie sie im den abhängigen Ansprüchen definiert sind.

Vorzugsweise handelt es sich dabei um ein orthopädisches Kleidungsstück, insbesondere orthopädisch indizierte einteilige Unterwäsche insbesondere für Patienten mit Osteoporose-Beschwerden, wobei die einteilige Unterwäsche aus einem Baumwoll- oder Kunstfasermaterial besteht und verschiedene Stretchzonen aufweist, die im angelegten Zustand eine Spannung aufweisen, dadurch gekennzeichnet, daß das Kleidungsstück im Rückenbereich mit einer oben oder unten geöffneten länglichen Tasche für eine eng, elastisch oder mit geringem Spiel in dieser gehaltenen, die Wirbelsäule abstützenden steifen Schiene versehen ist.

Nach einer bevorzugten Ausführung der Erfindung erstreckt sich die Tasche mit der Schiene sich vom Steißbein bis zum ersten Halswirbel. Die Tasche ist durch einen Reißverschluß, Druckknöpfe, Knöpfe oder einen Klettverschluß verschließbar.

Nach der bevorzugten Ausführung der Erfindung sind drei Stretchzonen vorhanden, wobei die erste Stretchzone quer um den Bauch verläuft und die zweite und dritte Stretchzone jeweils von der Brust über die Schulter in den Rücken reicht.

Die Schiene ist warmverformbar und kann vom Orthopäden angepaßt werden.

Nach einer bevorzugten Ausführung ist das Kleidungsstück in der Form einteiliger Unterwäsche mit Verschluß im Schrittbereich (sogenannter Body) geschnitten, wobei der Verschluß vorne supra-symphyser angeordnet ist.

Zusätzlich kann ein von dem vorderen Halsausschnitt abwärts gerichteter Knöpf-, Reiß-, Klett- und/oder Häkchen und Ösenverschluß vorhanden sein.

Nach einer anderen Ausführung der Erfindung ist das Kleidungsstück in der Form eines einteiligen Radfahrerdresses mit angesetzten Beinteilen geschnitten und der Verschluß ist ein von dem vorderen Halsausschnitt abwärts gerichteter Knöpf-, Reiß-, Klett- und/oder Häkchen und Ösenverschluß.

Bei beiden Ausführungen der Erfindung können im Schrittbereich weitere Öffnungen für hygienische Verrichtungen vorhanden sein.

Nach einer anderen Ausführung der Erfindung kann die Schiene aus mehreren formschlüssigen Gliedern bestehen, wobei Mittel vorhanden sind, die Glieder miteinander festzuziehen und so die steife Schiene zu bilden, sowie Mittel, die Versteifung aufzuheben. Die Mittel zum Festziehen sind beispielsweise einerseits wenigstens ein flexibles oder starres Band, das die Glieder durch miteinander fluchtende Bohrungen durch zieht und mit einem Ende an einem Ende des Schiene befestigt ist und bestehen andererseits aus einem sich an dem anderen Ende der schien abstützenden, mit dem anderen Ende des Bandes verbundenen Hebel, Exzenterhebel, Knebel, Schraubverschluß oder dergleichen.

Im folgenden wird die Erfindung anhand von Zeichnungen beispielhaft näher beschrieben. Dabei zeigen:
Fig. 1 eine Rückenansicht eines orthopädischen Kleidungsstücks;
Fig. 2 die Vorderansicht des Kleidungsstücks von Fig. 1;
Fig. 3 eine perspektivische Darstellung einer Schiene für das Kleidungsstück von Fig. 1 und 2.

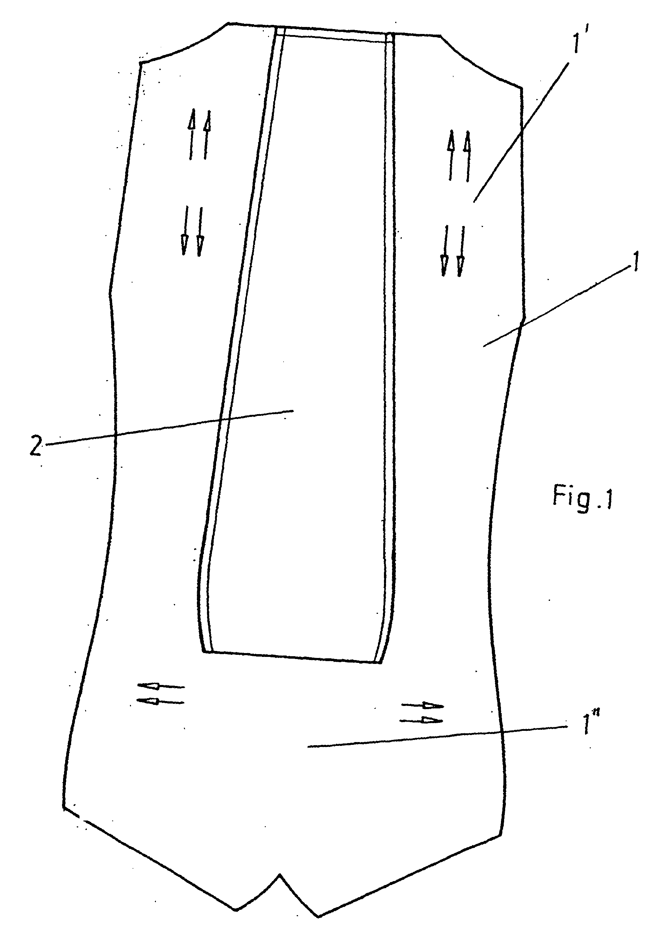

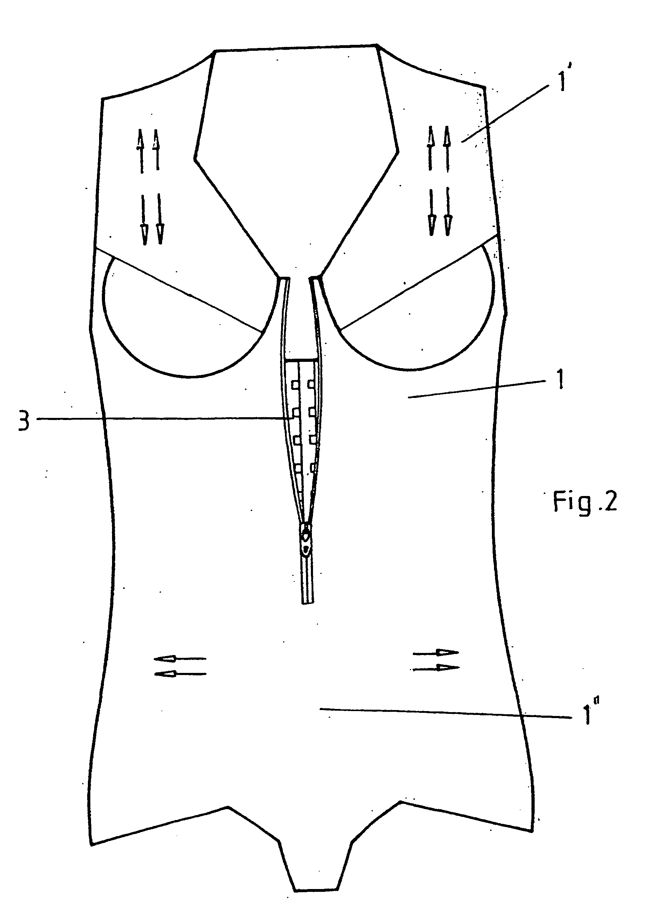

Das orthopädische Kleidungsstück 1 gemäß den Fig. 1 und 2, das wie ein herkömmlicher Body geschnitten ist, wobei die Darstellung schematisch ist, weist an seinem Rückenteil eine entlang der Wirbelsäule verlaufende, seitlich und oben geschlossene, unten jedoch offene aufgenähte Einstecktasche 2 auf, in die eine erfindungsgemäße orthopädische Schiene einzuführen ist, die die Wirbelsäule vom Steißbein bis zum ersten Halswirbel stützt. Entsprechend der Schienenform ist die Tasche 2 unten breiter und verjüngt sich in Halsrichtung. Die herausnehmbare Schiene ist in der Tasche mittels eines Klettverschlusses befestigt. Die offene Seite der Tasche kann auch mit einem Reißverschluß, Druckknöpfen oder dergleichen verschließbar sein. Vorne weist das Kleidungsstück 1 einen Reißverschluß 3 im Dékolleté auf. Das Kleidungsstück 1 weist weiter drei Stretchzonen auf, die durch elastisches Gewebe gebildet werden, wobei zur Versteifung mehrere Gewebelagen übereinander vorhanden sein können. Eine Stretchzone 1" verläuft horizontal um den Abdomen, während die beiden anderen Stretchzonen 1' vertikal vorne und hinten jeweils über die Schultern verlaufen.

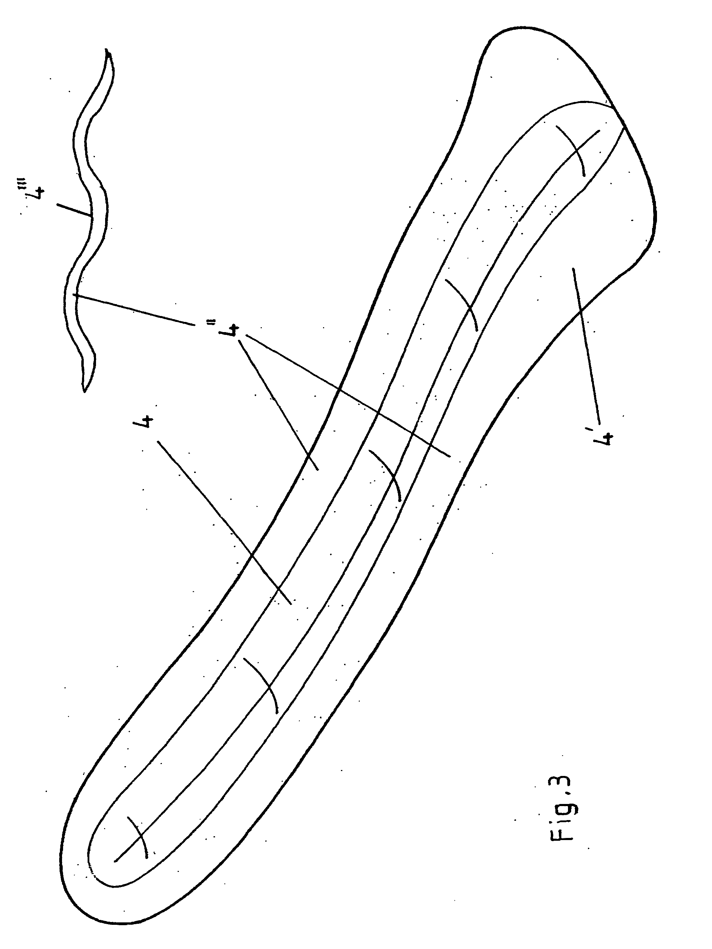

Die in Fig. 3 gezeigte Schiene 4, die in die Einstecktasche auf dem Rückenteil des orthopädischen Kleidungsstücks einzuführen ist, weist eine dem Verlauf der Wirbelsäule folgende, leicht gekrümmte Kontur auf und ist am unteren Ende 4' verbreitert ausgeführt. An den Seiten ist sie mit Verbreiterungen 4" versehen, die, wie aus dem rechts dargestellten Querschnitt versehen ist, zum Anliegen an den Körper des Patienten kommen, wobei zwischen Ihnen eine Aussparung 4'" vorhanden ist, die die Dornfortsätze der Wirbelsäule überbrückt. In der Praxis kann eine solche, zwar vorgeformte, jedoch warmverformbare Schiene, sehr flach und passend an die Wirbelsäule des Patienten anmodelliert werden. Durch die Wellenform von Aussparung 4"', einwärts gebogenen Verbreiterungen 4' und deren auslaufenden, wieder leicht auswärts gekrümmten Enden, wie in dem Querschnitt dargestellt, ist sie mechanisch sehr stabil, so daß eine flachere, anmodellierte Schiene die gleiche Stabilität aufweist, wie eine nicht anmodellierte vorgefertigte Schiene.

## Patentansprüche

1. Schiene für Bandagen, Mieder, Kleidungsstücke, Korsetts, Orthesen und dergleichen zur Behandlung der Osteoporose durch Stützung der Wirbelsäule,
wobei sich die Schiene (4) vom Steißbein bis zum ersten Halswirbel erstreckt,
**dadurch gekennzeichnet,**
**daß** die Schiene aus einem steifen Material besteht,
**daß** die Schiene eine Aussparung (4''') für die Dornfortsätze der Wirbelsäule aufweist und neben diesen gemäß dem Körper eines Patienten modelliert ist,
**daß** die Schiene im Bereich des Rumpfes einen vergrößerten Durchmesser ihres Querschnitts (4') aufweist,
und **daß** die Schiene wellenförmig dem Verlauf der Wirbelsäule angepaßt ist.

2. Orthopädisches Kleidungsstück (1), insbesondere orthopädisch indizierte einteilige Unterwäsche insbesondere für Patienten mit Osteoporose-Beschwerden,
wobei die einteilige Unterwäsche aus einem Baumwoll- oder Kunstfasermaterial besteht und verschiedene Stretchzonen aufweist, die im angelegten Zustand eine Spannung aufweisen,
**dadurch gekennzeichnet,**
**daß** das Kleidungsstück (1) im Rückenbereich mit einer oben oder unten geöffneten länglichen Tasche (2), welche sich entlang der Wirbelsäule des Trägers des Kleidungsstückes erstreckt und mit einer eng, elastisch oder mit geringem Spiel in dieser gehaltenen, die Wirbelsäule abstützenden steifen Schiene (4) nach Anspruch 1 versehen ist.

3. Orthopädisches Kleidungsstück nach Anspruch 2,
**dadurch** geke ennzeichnet,
daß die Tasche (2) mit der Schiene (4) sich vom Steißbein bis zum ersten Halswirbel erstreckt.

4. Orthopädisches Kleidungsstück nach Anspruch 2 oder 3,
**dadurch gekennzeichnet,**
**daß** die Tasche (2) durch einen Reißverschluß, Druckknöpfe, Knöpfe oder einen Klettverschluß verschließbar ist.

5. Orthopädisches Kleidungsstück nach einem der Ansprüche 2-4, **dadurch gekennzeichnet,**
**daß** drei Stretchzonen (1', 1") vorhanden sind,
wobei die erste Stretchzone quer um den Bauch verläuft und die zweite und dritte Stretchzone jeweils von der Brust über die Schulter in den Rücken reicht.

6. Orthopädisches Kleidungsstück nach einem der Ansprüche 2-5,
**dadurch gekennzeichnet,**
**daß** das Kleidungsstück (1) in der Form einteiliger Unterwäsche als sogenannter Body und mit Verschluß im Schrittbereich geschnitten ist.

7. Orthopädisches Kleidungsstück nach Anspruch 6,
**dadurch gekennzeichnet,**
**daß** der Verschluß vorne supra-symphyser angeordnet ist.

8. Orthopädisches Kleidungsstück nach Anspruch 6 oder 7,
**dadurch gekennzeichnet,**
**daß** zusätzlich ein von dem vorderen Halsausschnitt abwärts gerichteter Knöpf-, Reiß-, Klett- und/oder Häkchen und Ösenverschluß vorhanden ist.

9. Orthopädisches Kleidungsstück nach einem der Ansprüche 2 bis 5,
**dadurch gekennzeichnet,**
**daß** das Kleidungsstück in der Form eines einteiligen Radfahrerdresses mit angesetzten Beinteilen geschnitten ist,
und **daß** der Verschluß ein von dem vorderen Halsausschnitt abwärts gerichteter Knöpf-, Reiß-, Klett- und/oder Häkchen und Ösenverschluß ist.

10. Orthopädisches Kleidungsstück nach einem der Ansprüche 6 bis 9
**dadurch gekennzeichnet,**
**daß** im Schrittbereich weitere Öffnungen für hygienische Verrichtungen vorhanden sind.

11. Orthopädisches Kleidungsstück nach einem der Ansprüche 2 bis 10,
**dadurch gekennzeichnet,**
**daß** die Schiene aus mehreren formschlüssigen Gliedern besteht, wobei Mittel vorhanden sind, die Glieder miteinander festzuziehen und so die steife Schiene zu bilden, sowie Mittel, die Versteifung aufzuheben.

12. Orthopädisches Kleidungsstück nach Anspruch 11,
**dadurch gekennzeichnet,**
**daß** die Mittel zum Festziehen einerseits wenigstens ein flexibles oder starres Band sind, das die Glieder durch miteinander fluchtende Bohrungen durch zieht und mit einem Ende an einem Ende des Schiene befestigt ist,
und **daß** die Mittel zum Festziehen andererseits aus einem sich an dem anderen Ende der schien abstützenden, mit dem anderen Ende des Bandes verbundenen Hebel, Exzenterhebel, Knebel, Schraubverschluß oder dergleichen bestehen.

## Claims

1. A splint for bandages, bodices, garments, corsets, orthoses and the like for the treatment of osteoporosis by supporting the vertebral column, the splint (4) extending from the coccyx to the first cervical vertebra,
**characterised**
**in that** the splint is made of a stiff material,
**in that** the splint has a recess (4"') for the spinous processes of the vertebral column and alongside the said processes is moulded to conform to the body of a patient,
**in that** the splint has an enlarged diameter of its cross-section (4') in the region of the trunk,
and **in that** the splint is adapted in a wave-shape to the curvature of the vertebral column.

2. An orthopaedic garment (1), particularly an orthopaedically indicated one-piece undergarment especially for patients with osteoporosis pain, the one-piece undergarment being made of a cotton or synthetic fibre material and having various stretch zones which have a tension in the applied state,
**characterised**
**in that** the garment (1) is provided in the region of the back with an elongated pocket (2), open at the top or the bottom, which extends along the vertebral column of the wearer of the garment and comprises a stiff splint (4) according to Claim 1 held close-fittingly, flexibly or with a small amount of play in the said pocket and supporting the vertebral column.

3. An orthopaedic garment according to Claim 2,
**characterised**
**in that** the pocket (2) comprising the splint (4) extends from the coccyx to the first cervical vertebra.

4. An orthopaedic garment according to Claim 2 or 3,
**characterised**
**in that** the pocket (2) may be closed by means of a zip fastener, press studs, buttons or a hook and loop fastener.

5. An orthopaedic garment according to any one of Claims 2 - 4,
**characterised**
**in that** there are three stretch zones (1', 1"),
the first stretch zone extending transversely around the abdomen and the second and third stretch zone each extending from the chest over the shoulder to the back.

6. An orthopaedic garment according to any one of Claims 2 - 5,
**characterised**
**in that** the garment (1) is cut in the shape of a one-piece undergarment as a so-called body and with the fastener in the crotch region.

7. An orthopaedic garment according to Claim 6,
**characterised**
**in that** the fastener is arranged at the front over the symphysis.

8. An orthopaedic garment according to Claim 6 or 7,
**characterised**
**in that** there is additionally a button, zip, hook and loop fastener and/or hook and eye fastener extending downwardly from the front neckline.

9. An orthopaedic garment according to any one of Claims 2 to 5,
**characterised**
**in that** the garment is cut in the form of a one-piece cyclist's outfit with attached leg sections,
and **in that** the fastener is a button, zip, hook and loop fastener and/or hook and eye fastener extending downwardly from the front neckline.

10. An orthopaedic garment according to any one of Claims 6 to 9,
**characterised**
**in that** there are other openings in the crotch region for *excretion purposes*.

11. An orthopaedic garment according to any one of Claims 2 to 10,
**characterised**
**in that** the splint is composed of several form-fitting elements, means being present to tighten the elements together and thus form the stiff splint, and also means to discontinue the stiffening.

12. An orthopaedic garment according to Claim 11,
**characterised**
**in that** the means for tightening, on the one hand, are at least one flexible or rigid tape which passes through the elements through mutually aligned bores and one end of which is secured to one end of the splint,
and **in that** the means for tightening, on the other hand, consist of a lever, eccentric lever, toggle, screw closure or the like, supported on the other end of the splint and connected to the other end of the tape.

## Revendications

1. Attelle pour bandages, gaines, vêtements, corsets, orthèses et analogues destinée à traiter l'ostéoporose en supportant la colonne vertébrale, l'attelle (4) s'étendant du coccyx jusqu'à la première vertèbre cervicale,
**caractérisée en ce que** :
l'attelle est en un matériau rigide,
l'attelle comporte un creux (4"') destiné aux apophyses épineuses de la colonne vertébrale et en plus est modelée conformément au corps d'un patient,
l'attelle présente au niveau du torse une section (4') de diamètre agrandi, et
l'attelle est adaptée aux courbures de la colonne vertébrale.

2. Vêtement orthopédique (1), en particulier sous-vêtement d'une seule pièce à usage orthopédique notamment pour des patients souffrant d'ostéoporose, le sous-vêtement d'une seule pièce étant en laine ou en fibres synthétiques et comportant différentes zones étirables qui présente une tension lorsqu'il est mis,
**caractérisé en ce que** le vêtement (1) est doté d'une poche (2) allongée, ouverte en haut et en bas, qui s'étend le long de la colonne vertébrale du support du vêtement, et d'une attelle (4) selon la revendication 1 qui est maintenue étroitement, élastiquement ou avec un faible jeu dans cette poche et qui soutient la colonne vertébrale.

3. Vêtement orthopédique selon la revendication 2, **caractérisé en ce que** la poche (2) dotée de l'attelle (4) s'étend du coccyx jusqu'à la première vertèbre cervicale.

4. Vêtement orthopédique selon la revendication 2 ou 3, **caractérisé en ce que** la poche (2) peut être fermée par une fermeture à glissière, des boutons à pression, des boutons ou une fermeture "Velcro".

5. Vêtement orthopédique selon l'une des revendications 2 à 4, **caractérisé en ce qu'**il est prévu trois zones étirables (1', 1"), la première zone étirable s'étendant transversalement autour du ventre et les deuxième et troisième zones étirables s'étendant de la poitrine jusqu'au dos en passant par-dessus l'épaule.

6. Vêtement selon l'une des revendications 2 à 5, **caractérisé en ce que** le vêtement (1) est coupé sous la forme d'un sous-vêtement d'une seule pièce de type justaucorps et est doté d'une fermeture au niveau de l'entrejambe.

7. Vêtement orthopédique selon la revendication 6, **caractérisé en ce que** la fermeture est placée en avant dans la région suprasymphysaire.

8. Vêtement orthopédique selon la revendication 6 ou 7, **caractérisé en ce qu'**il est prévu en plus une fermeture "Velcro", à boutons, et/ou à agrafes et oeillets dirigée vers le bas depuis l'encolure avant.

9. Vêtement orthopédique selon l'une des revendications 2 à 5,
**caractérisé en ce que**
le vêtement est coupé sous la forme d'une tenue de cycliste d'une seule pièce à laquelle sont ajoutées des pièces pour les jambes, et **en ce que**
la fermeture est une fermeture "Velcro", à boutons, et/ou à agrafes et oeillets.

10. Vêtement orthopédique selon l'une des revendications 6 à 9, **caractérisé en ce que** des ouvertures supplémentaires pour les besoins hygiéniques sont ménagées au niveau de l'entrejambe.

11. Vêtement orthopédique selon l'une des revendications 2 à 10, **caractérisé en ce que** l'attelle est constituée de plusieurs éléments à verrouillage de forme, des moyens étant prévus pour bloquer les éléments entre eux et pour former l'attelle rigide ainsi que des moyens pour supprimer le raidissement.

12. Vêtement orthopédique selon la revendication 11, **caractérisé en ce que** les moyens de blocage sont constitués d'une part au moins par une bande flexible ou rigide qui traverse les éléments par des orifices alignés et qui est fixée par une extrémité à une extrémité de l'attelle, et **en ce que** les moyens de blocage sont constitués d'autre part d'un levier, d'un levier excentrique, d'une manette, d'une fermeture à vis ou analogue qui est relié à l'autre extrémité de la bande et qui supporte l'autre extrémité de l'attelle.
